# EUROPÄISCHE PATENTSCHRIFT

(11) **EP 1 531 891 B1**
(45) Veröffentlichungstag und Bekanntmachung des Hinweises auf die Patenterteilung: **25.11.2009**
(21) Anmeldenummer: 03792320.8
(22) Anmeldetag: 14.08.2003
(51) Int. Cl.: A61M 16/00, A61M 16/10

(54) **VORRICHTUNG ZUR BEEINFLUSSUNG VON GASFLÜSSEN**
DEVICE FOR INFLUENCING GAS FLOWS
DISPOSITIF DESTINE A INFLUENCER DES FLUX GAZEUX

(30) Priorität: 19.08.2002 DE 10238683
(43) Veröffentlichungstag der Anmeldung: 25.05.2005
(60) Teilanmeldung: 08004679.0
(73) Patentinhaber: Rist, Max, 85276 Pfaffenhofen (DE)
(72) Erfinder: Rist, Max, 85276 Pfaffenhofen (DE)
(74) Vertreter: Liebl, Thomas
(86) Internationale Anmeldenummer: PCT/EP2003/009041
(87) Internationale Veröffentlichungsnummer: WO 2004/018026

(56) Entgegenhaltungen:
- EP-A- 0 972 534
- DE-A- 4 319 458
- GB-A- 1 190 441
- US-A- 5 320 093
- PATENT ABSTRACTS OF JAPAN vol. 017, no. 574 (M-1498), 19. Oktober 1993 (1993-10-19) & JP 05 164359 A (MATSUSHITA ELECTRIC IND CO LTD), 29. Juni 1993 (1993-06-29)

## Beschreibung

Bei pulmonal erkrankten Patienten werden zur Therapie häufig Aerosole appliziert. Besondere Schwierigkeiten stellen bei der Anwendung von Aerosolen die beatmeten Patienten dar. Es werden entweder druckluftabhängige Düsenvernebler oder kontinuierlich Aerosol produzierende Vernebler wie z. B. Ultaschallvernebler bzw. piezoelektische Vernebler eingesetzt. Die Düsenvernebler werden normalerweise in der gemeinsamen Endstrecke für In- und Exspiration der Beatmungsschläuche kurz vor dem Tubus platziert und über die Beatmungsgeräte gesteuert, so dass sie nur während der Inspiration aktiv sind. Allerdings ist der Vernebler bis zum Ende der Inspiration tätig, sodass im gesamten Schlauchsystem distal des Verneblers mit Aerosol angereichertes Gas steht, so dass auch das Totraumvolumen mit Aerosol versehen wird. So kann zwar zwischen dem In- bzw. Exspirationsschenkel und Tubus ein Bakterienfilter platziert werden, der seinen Sinn auch in der Befeuchtung der Atemwege hat. Die starke Befeuchtung der ungenützten Gassäule (Totraumvolumen) vor allem mit großen Aerosolpartikeln aber bewirkt, dass sich der Filter mit Flüssigkeit voll saugt und damit einerseits an antibakterieller Wirkung verliert und andererseits der Widerstand des Filters so groß wird, dass er sich verschließt.

Die kontinuierlichen Vernebler wie z. B. Ultraschallvernebler bzw. piezoelektischen Vernebler stellen kleinere Partikel (durchschnittlichen Größe unter 7µm) her, die besser an den Einsatzort gelangen, verbunden mit geringeren Nebenwirkungen und geringeren Medikamentenkosten. Allerdings können diese Geräte nur im Inspirationsschenkei eines Beatmungsgerätes platziert werden, da sie kontinuierlich Aerosol produzieren und so in der gemeinsamen Endstrecke sowohl in der Inspiration als auch in der Exspiration Aerosol zum bzw. vom Patienten weg transportiert würde. Ein Filter würde sich so vollsaugen und an Wirksamkeit verlieren, bzw. sich verschließen. Durch den Verzicht auf Filter erfolgt keine ausreichende Atemluftbefeuchtung, so dass ein Atemluftbefeuchter verwendet werden muss, der neben den Kosten auch ein hygienisches Problem (nosokomiale Pneumonie) darstellt.

Die GB-A-1 190 441 offenbart eine Vorrichtung zur Richtungslenkung von Gasflüssen. Die Vorrichtung weist ein Beatmungsgerät, einen Vernebler und eine ventilkontrollierte Bypasseinrichtung auf, bei der sich die Bypasseinrichtung nicht am Vernebler befindet. Vielmehr handelt es sich hier um ein herkömmliches Kreisteil mit einem Hin- und einem Rückatmungsbereich, wobei im Bereich des Rückatmungsbereiches eine Gasmesseinrichtung vorgesehen ist, die mittels eines Ventils zu Gasmessungszwecken zugeschalten werden kann. In diesem zugeschalteten Zustand der Gasmesseinrichtung muss aber im sich stromabwärts des Ventils anschließenden Rückatmungsteilbereich aufgrund der Anordnung des weiterhin betätigten Faltenbalges eine Strömung ermöglicht werden, wozu die Bypassleitung vorgesehen ist. Diese Bypassleitung hat somit lediglich den Zweck, im Falle der Zuschaltung des Gasmessgerätes den Strömungsfluss bzw. die Funktionsweise der Vorrichtung insgesamt aufrechtzuerhalten.

Der Erfindung liegt das Problem zugrunde, eine Vorrichtung in Verbindung mit Aerosol produzierenden Verneblern zu schaffen, mit der die oben beschriebenen Probleme vermieden werden können.

Dieses Problem wird durch die im Patentanspruch 1 aufgeführten Merkmale, mit Hilfe der Vorrichtung zur Richtungslenkung von Gasflüssen bei Verneblern in Hin- und Rückatmungsbereichen eines Beatmungsgerätes, wobei eine Bypasseinrichtung vorgesehen ist, die beim Fluss des Gases in die eine Richtung das Aerosol mitnimmt, beim Fluss in die andere Richtung über einen Bypass am Vernebler vorbeigeführt wird, gelöst.

Die Erfindung ermöglicht die Platzierung von Verneblern (jeder Vernebler ist verwendbar) zwischen Filter und Tubus ohne Atemluftbefeuchter an der gemeinsamen Endstrecke mit dem Vorteil des geringeren Verbrauchs an Medikamenten, weniger Nebenwirkungen durch die applizierten Medikamente und der längeren Haltbarkeit der Filter.

Dies geschieht, indem im Sinne der Erfindungsidee des Patentanspruchs 1 über Ventile die Atemluft bei der Inspiration über den Vernebler geführt wird, bei der Exspiration durch sich schließende Ventile (passiv oder aktiv gesteuert) die Ausatemluft über einem Bypass vorbei am Vernebler geleitet wird. Um dies zu ermöglichen können die Ventile an verschiedenen Stellen in dieser Gerätschaft platziert werden. Auch besteht in dieser Vorrichtung die Möglichkeit mit Zusatzeinrichtungen (z.B. durch Ventilatoren) die Verteilung des Aerosols zu optimieren.

Die Erfindung wird nachfolgend anhand einer Zeichnung näher erläutert.

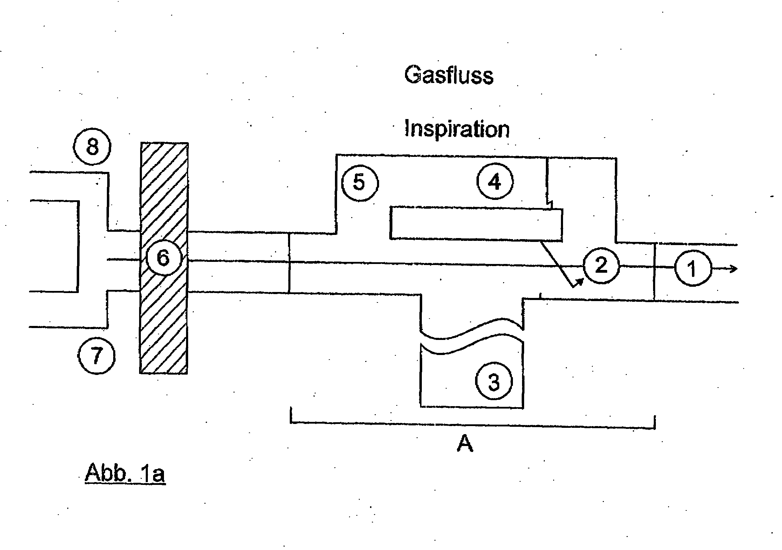

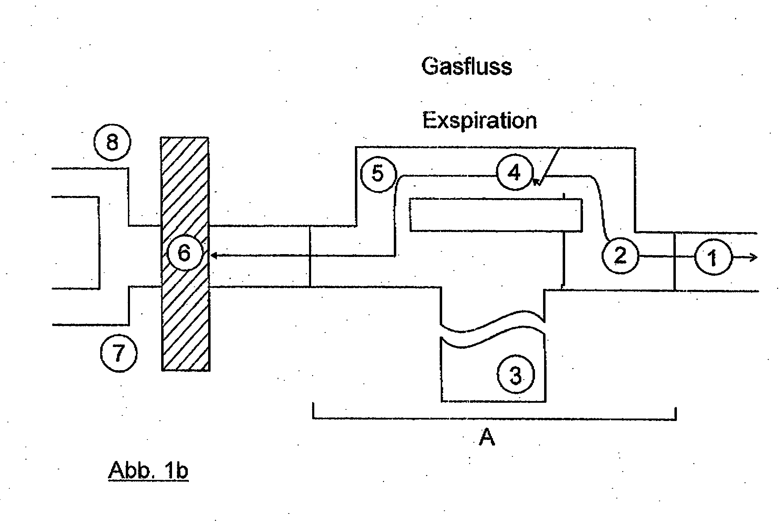

Es zeigen die Abbildungen 1 die Erfindung gemäß einer Ausführungsform nach Patentanspruch 1 (A). Sie ermöglicht eine Platzierung von Verneblern (jeder Vernebler ist verwendbar) (3) zwischen Filter (6) und Verbindungsstück zum Tubus (7) ohne Atemluftbefeuchter an der gemeinsamen Endstrecke (1). Dies geschieht indem über Ventile (2, 4) (es können sämtliche möglichen Ventilarten sein) die Atemluft bei der Inspiration über den Vernebler geführt wird, indem sich das Ventil 2 öffnet und das Ventil 4 schließt (passiv oder aktiv gesteuert) (Abb. 1 a). Bei der Exspiration schließt sich Ventil 2 und öffnet sich Ventil 4 (passiv oder aktiv gesteuert), so dass die Ausatemluft über einem Bypass (5) am Vernebler vorbei geleitet wird (Abb. 1b). Um dies zu ermöglichen können die Ventile an verschiedenen Stellen in dieser Gerätschaft platziert werden (Inspirationsventil: zwischen Ein- und Auslass des Bypasses; Exspirationsventil: im ganzen Bypass). Der lnspirationsschenkel des Beatmungsschlauchs wird durch die Nummer 7 dargestellt, der Exspirationsschenkel durch die Nummer 8.

## Patentansprüche

1. Vorrichtung zur Richtungslenkung von Gasflüssen eines Beatmungsgerätes,
mit einem Beatmungsgerät,
mit einem im Hin- und Rückatmungsbereich des Beatmungsgerätes angeordneten, Aerosol produzierenden Vernebler (3), und
mit einer Bypasseinrichtung,
**dadurch gekennzeichnet,**
**dass** die Bypassanordnung so ausgebildet ist, dass das Gas beim Fluss in die eine Richtung über den Vernebler (3) geführt wird zur Mitnahme von Aerosol und dass das Gas beim Fluss in die andere Richtung über einen Bypass (5) am Vernebler (3) vorbeigeführt wird.

## Claims

1. Device for controlling the direction of gas flows of a respiratory appliance,
with a respiratory appliance,
with an atomizer (3) arranged in the breathing and rebreathing area of the respiratory appliance and producing an aerosol, and
with a bypass arrangement,
**characterized in that**
the bypass arrangement is designed such that the gas is guided across the atomizer (3) in order to take up aerosol when flowing in one direction and **in that** the gas is guided past the atomizer (3) through a bypass (5) when flowing in the other direction.

## Revendications

1. Dispositif destiné à diriger les flux gazeux d'un respirateur, comportant
- un respirateur,
- un nébuliseur (3) produisant de l'aérosol, agencé dans la zone d'inhalation et de réinhalation du respirateur, et
- un dispositif bypass,
**caractérisé**
**en ce que** l'ensemble bypass est réalisé de telle sorte que le gaz est conduit, en cas d'écoulement dans un sens, par le biais du nébuliseur (3) pour entraîner l'aérosol, et en ce que le gaz passe, en cas d'écoulement dans l'autre sens, devant un nébuliseur (3) par le biais d'un bypass (5).
